# EUROPEAN PATENT SPECIFICATION

(11) **EP 1 575 671 B1**
(45) Date of publication and mention of the grant of the patent: **18.01.2012**
(21) Application number: 03767578.2
(22) Date of filing: 20.11.2003
(51) Int. Cl.: A61P 3/04, A61K 31/353, A61K 31/12, A23L 1/30

(54) **NUTRACEUTICAL COMPOSITIONS COMPRISING EPIGALLOCATECHIN GALLATE AND RASPBERRY KETONE**
FUNCTIONAL-FOOD-ZUSAMMENSETZUNGEN ENTHALTEND EPIGALLOCATECHIN UND HIMBEERKETON
NOUVELLES COMPOSITIONS NUTRACEUTIQUES COMPRENANT DU GALLATE D'EPIGALLOCATECHINE ET DE LA CETONE DE FRAMBOISE

(30) Priority: 28.11.2002 EP 02026575
(43) Date of publication of application: 21.09.2005
(73) Proprietor: DSM IP Assets B.V., 6411 TE Heerlen (NL)
(72) Inventor: RAEDERSTORFF, Daniel, F-68720 Flaxlanden (FR); WEBER, Peter, D- 79379 Müllheim (DE); WOLFRAM, Swen, 79761 Waldshut-Tiengen (DE)
(74) Representative: Grossner, Lutz
(86) International application number: PCT/EP2003/012975
(87) International publication number: WO 2004/047926

(56) References cited:
- EP-A- 0 847 698
- WO-A-01/60319
- WO-A-02/072086
- PATENT ABSTRACTS OF JAPAN vol. 2000, no. 09, 13 October 2000 (2000-10-13) -& JP 2000 169325 A (KANEBO LTD), 20 June 2000 (2000-06-20)

## Description

The present invention relates to novel nutraceutical compositions comprising epigallocatechin gallate (hereinafter : EGCG) and raspberry ketone (4-(4-hydroxyphenyl)-2-butanone, hereinafter : RK). More specifically, the invention relates to novel nutraceutical compositions for the treatment or prevention of obesity, or conditions associated with obesity such as non-insulin-dependent diabetes mellitus (NIDDM, type II) and syndrome X.

The composition is particularly intended for the treatment or prevention of obesity, and for the prevention of NIDDM in those individuals that are at high risk, e.g., that are overweight or have impaired glucose tolerance.

WO 02/072086 A discloses the use of EGCG in the prevention and treatment of, inter alia, obesity. WO 01/60319 A discloses the use of, inter alia, EGCG for reducing food intake in a subject. Patent Abstracts of Japan vol. 2000, no 09 (JP 2000 169325 A) describes the use of, inter alia, raspberry ketone as a promoter for reducing fatty tissue, in particular in skin cosmetics.

The composition of the invention comprises a combination of EGCG and RK, which have different mechanisms of action on glucose and fat uptake as well as excretion, hepatic glucose production, and energy expenditure. Thus, the combination is providing additive and/or synergistic effects for the treatment or prevention of obesity.

The term nutraceutical as used herein denotes usefulness in both the nutritional and pharmaceutical field of application. Therefore, the novel nutraceutical composition can be used as a supplement to food and beverages, and as pharmaceutical formulation for enteral or parenteral application, which may be solid formulations such as capsules or tablets, or liquid formulations such as solutions or suspensions. As will be evident from the foregoing, the term nutraceutical composition also comprises food and beverages containing EGCG and RK as well as supplement compositions containing both active ingredients.

Even though awareness of the association between obesity and health problems is longstanding the prevalence of obesity has grown to epidemic proportions during the last few decades. Currently, more than 50% of the US population are overweight and approximately 20% are considered to be obese or extremely overweight. The prevalence of obesity is still increasing rapidly not only in industrialized countries. Obesity will become one of the most important public health problems in nonindustrialized countries, particularly in those undergoing economic transition. The World Health Organization (WHO) has estimated that in 2025, approximately 300 million people will be obese.

Obesity is the most important risk factor for the onset of NIDDM. Moreover, being moderately overweight is also closely associated to the onset of NIDDM. The WHO calculated that the prevalence of patients with NIDDM could be reduced by as much as 64% in US men and 74% in US women, if there were no overweight and obese subjects. NIDDM is associated with increased risk for mirco- and macrovascular diseases, including nephropathy, and neuropathy.

Obesity has the strongest impact on cardiovascular risk profile among all other risk factors. Obese subjects are at high risk for increased blood pressure and unfavorable lipid profile such as decreased high density lipoprotein (HDL) cholesterol levels and increased low density lipoprotein (LDL) cholesterol as well as triglyzeride levels. Weight loss was demonstrated to reduce blood pressure and to improve lipid levels and a high body weight is associated with increased cardiovascular mortality.

Furthermore, obesity is an established risk factor for endometrial cancer. There is evidence for a possible relationship between obesity and breast, kidney, colon, prostate, and gallbladder cancer. Not only that obesity causes cancer, it might also influence cancer detection due to a lack in compliance for screening programs and also due to detection problems caused by adipose tissue. In addition, obesity is one of the most important risk factors for osteoarthritis in knee and hip joints and associations with herniated lumbar intervertebral disc, lower back pain, and chronic neck pain have been suggested. Obstructive sleep apnea and shortness of breath are typical respiratory consequences of obesity.

Thus, the treatment or prevention of obesity would reduce the prevalence of a variety of chronic diseases and acute adverse events. Typically, the treatment of obesity involves dietary and lifestyle interventions. However, the compliance of patients to such programs is low. Patients commonly do not recognize obesity as a disease and are therefore less likely to respond to lifestyle changes. Furthermore, even multidisciplinary approaches including dietary measure, increase in physical activity and behavior modification do not provide a satisfactory success rate, particularly if long-term results are considered.

Therefore, the use of weight lowering agents has been proposed as a more effective treatment choice for obesity. However, the development of drugs like amphetamines was not only disappointing in terms of efficacy but turned out to cause a number of partially severe adverse effects, which finally led to the withdrawal of most of these compounds. Serotoninergic drugs such as fenfluramine and dexfenfluramine were also associated with severe adverse effects and as a consequence withdrawn from the market. Other treatments like the noradrenergic/serotoninergic drug sibutramine have side effects ranging from dry mouth, insomnia, anorexia, constipation, and increase in heart rate as well as blood pressure. Lipase inhibitors like orlistat cause fecal urgency, oily spotting, fatty stool, flatus, discharge, increased defecation and fecal incontinence, occurring with a frequency of 10 to 30%.

These facts illustrate that there is need for a safe and effective nutritional supplement with minimal side effects for the treatment and prevention of obesity. Obese subjects are interested in substances that they consider being natural or plant derived food ingredients without major side effects. These compounds could be used as adjuvant treatment together with dietary intervention and increased physical activity. Furthermore, obesity is a complex and multifactorial disease and thus, a combination therapy is an attractive and feasible approach to reduce the body weight of a variety of obese patients.

Epigallocatechin gallate (EGCG) is the major catechin found in green tea. The beneficial health effects of green tea have been mainly attributed to the catechins. In mice, tea catechins reduced diet-induced weight gain, visceral fat mass, as well as plasma leptin, triglyceride, and glucose levels. Tea catechins are also known to increase energy expenditure in rats. In humans, tea catechins have been shown to reduce body weight, visceral fat mass, and plasma cholesterol, insulin, and glucose levels. Green tea extract was shown to significantly increase energy expenditure and fat oxidation in healthy men. Furthermore, it was shown in brown adipose tissue of rats that EGCG stimulates metabolic activity and oxygen consumption. Additionally, several animal studies demonstrated that catechins inhibited cholesterol absorption and lowered plasma cholesterol levels. In turn, epicatechins increase the fecal excretion of cholesterol and total lipids. Therefore, EGCG has an antiobesity effect, through a stimulation of thermogenesis and/or an altered fat absorption.

Raspberry ketone (4-(4-hydroxyphenyl)-2-butanone, RK) is one of the character impact components of raspberry flavor. The compound has been identified as the free ketone and as glucoside in the berries. The compound accumulates rapidely during the ripening of rasberry fruits. RK or its glucoside are not only found in the raspberry fruit but also in rhubarb roots, cranberries and the needles of pine.

The nutraceutical composition of the present invention contains EGCG in an amount sufficient to administer to a subject a dosage from about 0.01 mg to about 60 mg per kg body weight per day, preferably from about 0.1 mg to about 10 mg per kg body weight per day. Thus, if the nutraceutical composition is a food or beverage the amount of EGCG contained therein is suitably in the range from about 0.3 mg per serving to about 1250 mg per serving. If the nutraceutical composition is a pharmaceutical formulation such formulation may contain from about 1 mg to about 4000 mg per solid dosage unit, e.g., per capsule or tablet, or a corresponding dosage in a liquid formulation, or from about 1 mg per daily dose to about 4000 mg per daily dose. In a preferred aspect of the invention, the nutraceutical composition of the present invention further contains rasberry ketone (RK). The amount of RK in the composition may be such to provide a daily dosage from about 0.01 mg per kg body weight to about 60 mg per kg body weight of the subject to which it is to be administered. A food or beverage suitably contains about 0.3 mg per serving to about 1250 mg per serving of RK. If the nutraceutical composition is a pharmaceutical formulation such formulation may contain RK in an amount from about 1 mg to about 4000 mg per dosage unit, e.g., per capsule or tablet, or from about 1 mg per daily dose to about 4000 mg per daily dose of a liquid formulation.

Dosage ranges (for a 70 kg person) : Epigallocatechin gallate (EGCG) : 1 to 4000 mg/day; Raspberry ketone (RK) : 1 to 4000 mg/day.

The following examples illustrate the invention further.

### A. Pharmaceutical compositions may be prepared by conventional formulation procedures using the ingredients specified below:

### Example 1

### Soft gelatin capsule

Soft gelatin capsules can be prepared by conventional procedures using ingredients specified below:

| | |
|---|---|
| Epigallocatechin gallate (EGCG) | 100 mg |
| Raspberry ketone (RK) | 100 mg |

Other ingredients: glycerol, water, gelatine, vegetable oil

### Example 2

### Hard gelatin capsule

Hard gelatin capsules are prepared by conventional procedures using ingredients specified below:

| | |
|---|---|
| Epigallocatechin gallate (EGCG) | 100 mg |
| Raspberry ketone (RK) | 100 mg |

Other ingredients:
Fillers: lactose or cellulose or cellulose derivatives q.s.
Lubricant: magnesium sterate if necessary (0.5%)

### Example 3

**Tablet**

| | |
|---|---|
| Tablets can be prepared by conventional procedures using ingredients specified below: | |
| Epigallocatechin gallate (EGCG) | 50 mg |
| Raspberry ketone (RK) | 50 mg |

Other ingredients: microcrystalline cellulose, silicone dioxide (siO2), magnesium stearate, crosscarmellose sodium

### B. Food items may be prepared by conventional procedures using ingredients specified below:

### Example 4

### Soft Drink with 30% juice

| | |
|---|---|
| Typical serving: | 240 ml |

### Active ingredients:

| | |
|---|---|
| Epigallocatechin gallate (EGCG): | 0.3-1250 mg/ per serving |
| Raspberry ketone (RK): | 0.3-1250 mg/ per serving |

### 1. A Soft Drink Compound is prepared from the following ingredients :

### Juice concentrates and water soluble flavours

| | [g] |
|---|---|
| 1.1 Orange concentrate | |
| 60.3 °Brix, 5.15% acidity | 657.99 |
| Lemon concentrate | |
| 43.5 °Brix, 32.7% acidity | 95.96 |
| Orange flavour, water soluble | 13.43 |
| Apricot flavour, water soluble | 6.71 |
| Water | 26.46 |
| 1.2 Color | |
| β-Carotene 10% CWS | 0.89 |
| Water | 67.65 |
| 1.3 Acid and Antioxidant | |
| Ascorbic acid | 4.11 |
| Citric acid anhydrous | 0.69 |
| Water | 43.18 |
| 1.4 Stabilizers | |
| Pectin | 0.20 |
| Sodium benzoate | 2.74 |
| Water | 65.60 |

| 1.5 Oil soluble flavours | |
|---|---|
| Orange flavour, oil soluble | 0.34 |
| Orange oil distilled | 0.34 |

### 1.6 Active ingredients

Active ingredients EGCG and RK in the concentrations mentioned above.

Fruit juice concentrates and water soluble flavours are mixed without incorporation of air. The color is dissolved in deionized water. Ascorbic acid and citric acid is dissolved in water. Sodium benozoate is dissolved in water. The pectin is added unter stirring and dissolved while boiling. The solution is cooled down.Orange oil and oil soluble flavours are premixed. The active ingredients as mentioned under 1.6 are dry mixed and then stirred preferably into the fruit juice concentrate mixture (1.1).

In order to prepare the soft drink compound all parts 3.1.1 to 3.1.6 are mixed together before homogenising using a Turrax and then a high-pressure homogenizer (p₁ = 200 bar, P₂ = 50 bar).

### II. A Bottling Syrup is prepared from the following ingredients:

| | [g] |
|---|---|
| Softdrink compound | 74.50 |
| Water | 50.00 |
| Sugar syrup 60° Brix | 150.00 |

The ingredients of the bottling syrup are mixed together. The bottling syrup is diluted with water to 1l of ready to drink beverage.

### Variations :

Instead of using sodium benzoate, the beverage may be pasteurised. The beverage may also be carbonised.

### Example 5

### 5 Cereal Bread

| | |
|---|---|
| Typical serving: | 50 g |

### Active ingredients:

| | |
|---|---|
| Epigallocatechin gallate (EGCG): | 0.3-1250 mg/ per serving |
| Raspberry ketone (RK): | 0.3-1250 mg/ per serving |

### Other components:

| | [%] |
|---|---|
| 5 cereal flour | 56.8 |
| Water | 39.8 |
| Yeast | 2.3 |
| Salt | 1.1 |

The yeast is dissolved in a part of the water. All ingredients are mixed together to form a dough. Salt is added at the end of the kneading time. After fermentation, the dough is reworked and divided before a loaf is formed. Before baking, the surface of the loaf is brushed with water and sprinkled with flour.

### Procedure:

| Kneading: | |
|---|---|
| Spiral kneading system | 4 min 1^{st} gear |
| | 5 min 2^{nd} gear |
| Dough proofing: | 60 min |
| Dough temperature: | 22 - 24 °C |
| Proofing time: | 30 min |

| Baking: | |
|---|---|
| Oven: | Dutch type oven |
| Baking temperature: | 250/220 °C |
| Baking time: | 50 - 60 min |

### Example 6

### Cookies Type Milano

| | |
|---|---|
| Typical serving: | 30 g |

### Active ingredients:

| | |
|---|---|
| Epigallocatechin gallate (EGCG): | 0.3-1250 mg/ per serving |
| Raspberry ketone (RK): | 0.3-1250 mg/ per serving |
| Other components: | [g] |
| Wheat Flour, type 550 | 41.0 |
| Sugar | 20.5 |
| Fat/Butter | 20.5 |
| Whole egg (liquid) | 18.0 |
| Lemon Flavour | q.s. |
| Baking agent | q.s. |

All ingredients are added slowly under mixing to form a sweet short pastry.

Afterwards, the pastry is kept cool (4°C) for at least 2 hours before flattening the pastry to a thickness of approx. 5 mm. Pieces are cut out and brushed with egg yolk on the surface before baking.

### Baking:

Oven: fan oven
Baking temperature: 180 °C
Baking time: 15 min

### Example 7

### Toast

| | |
|---|---|
| Typical serving: | 100 g |

| Active ingredients: | |
|---|---|
| Epigallocatechin gallate (EGCG): | 0.3-1250 mg/ per serving |
| Raspberry ketone (RK): | 0.3-1250 mg/ per serving |

| Other components: | [%] |
|---|---|
| Wheat Flour, type 550 | 55.4 |
| Water | 33.2 |
| Yeast | 2.8 |
| Salt | 1.1 |
| Fat/Butter | 5.5 |
| Malt | 0.6 |
| Emulsifier baking agent | 1.4 |

The yeast is dissolved in a part of the water. All ingredients are mixed together to form a dough. Salt is added at the end of the kneading time. Afterwards, the dough is reworked, divided and placed in a baking tin for fermentation. After baking, the loaf is unmoulded directly.

### Procedure:

| Kneading | | |
|---|---|---|
| Spiral kneading system | | 5 - 6 min 1^{st} gear |
| | | 3 - 4 min 2^{nd} gear |
| Dough proofing: | none | |
| Dough temperature: | 22 - 24 °C | |
| Proofing time: | 40 min | |

| Baking: | | |
|---|---|---|
| Oven: | Dutch type oven | |
| Baking temperature: | 220 °C | |
| Baking time: | 35 - 40 min | |

### Example 8

| | |
|---|---|
| Yoghurt - set type; 3.5% fat | |
| Typical serving: | 225 g |

### Active ingredients:

| | |
|---|---|
| Epigallocatechin gallate (EGCG): | 0.3-1250 mg/ per serving |
| Raspberry ketone (RK): | 0.3-1250 mg/ per serving |

| Other components: | [%] |
|---|---|
| Full fat milk (3.8% fat) | 90.5 |
| Skimmed milk powder | 2.0 |
| Sugar | 5.0 |
| Culture | 2.5 |

The milk is heated to 35 °C before addition of milk powder, stabiliser, sugar and active ingredients. This mixture is heated to 65 °C to dissolve all ingredients. Then the mixture is homogenized in a high-pressure homogenizer (p₁ = 150 bar, p₂ = 50 bar) at 65 °C. This emulsion is then pasteurised at 80 °C for 20 minutes. After cooling to 45 °C natural yoghurt/culture is added and mixed. Then this mixture is filled into cups and fermented at 45 °C for 3-4 hours until a pH of 4.3 is reached and then stored at 4 °C.

### Example 9

Yoghurt - stirred type; 3.5% fat

| | |
|---|---|
| Typical serving: | 225 g |

### Active ingredients:

| | |
|---|---|
| Epigallocatechin gallate (EGCG): | 0.3-1250 mg/ per serving |
| Raspberry ketone (RK): | 0.3-1250 mg/ per serving |

| Other components: | [%] |
|---|---|
| Full fat milk (3.8% fat) | 90.2 |
| Skimmed milk powder | 2.0 |
| Stabiliser | 0.3 |
| Sugar | 5.0 |
| Culture | 2.5 |

The milk is heated to 35 °C before addition of milk powder, stabiliser, sugar and active ingredients. This mixture is heated to 65 °C to dissolve all ingredients before homogenisation in a high-pressure homogenizer (p₁ = 150 bar, p₂ = 50 bar) at 65°C. This emulsion is then pasteurised at 80 °C for 20 minutes. After cooling to 45 °C natural yoghurt/culture is added and mixed, followed by fermentation at 45 °C for 3-4 hours until a pH of 4.3 is reached. After cooling and stirring vigorously, the yoghurt is filled in cups and stored at 4 °C.

### Example 10

Ice cream; 8% fat

| | |
|---|---|
| Typical serving: | 85 g |

### Active ingredients:

| | |
|---|---|
| Epigallocatechin gallate (EGCG): | 0.3-1250 mg/ per serving |
| Raspberry ketone (RK): | 0.3-1250 mg/ per serving |

| Other components: | [g] |
|---|---|
| Milk (3.7% fat) | 600.00 |
| Cream (35% fat) | 166.00 |
| Skim milk powder | 49.10 |
| Sugar | 109.00 |
| Glucose syrup 80% | 70.00 |
| Ice cream stabiliser | 5.00 |
| Flavor | q.s. |

| | |
|---|---|
| Color | q.s |

Sugar, skim milk powder and stabiliser are added to the milk and cream, mixed and heated to 45 °C. Then the colour as stock solution and the glucose syrup is added as well as the active ingredients. The mix is heated up and pasteurized (20 min, 80 °C). Then a homogenization step takes place. Afterwards the mix is cooled down under constant stirring and the flavour is added at 5°C. The mix maturated at 5 °C during at least 4 h and then passed through an the ice cream machine (overrun ca. 100%). The ice cream is filled into cups and stored at -20 to -30 °C.

### Example 11

### Wine gums

### Active ingredients:

| | |
|---|---|
| Epigallocatechin gallate (EGCG): | 0.3-1250 mg/ per 30 g |
| Raspberry ketone (RK): | 0.3-1250 mg/ per 30 g |

| Other components: | [g] |
|---|---|
| Gelatine 200 Bloom | 80.0 |
| Water I | 125.0 |
| Sugar crys. | 290.0 |
| Water II | 120.0 |
| Glucose-syrup DE 38 | 390.0 |
| Citric acid | 10.0 |
| Flavour | 2.0 |
| Colour | q.s. |
| Yield ca | 1000.0 |

Disperse gelatine in water I, stir and dissolve by heating over a stream bath or using a microwave. Mix sugar with water II and bring to boiling until a clear solution is obtained. Remove from heat source. Mix with glucose syrup while dissolved sugar solution is still hot. Slowly add the gelatine solution. Let rest until foam on surface can be removed and 60-65°C is reached. Add flavour, citric acid and the colour solution as well as active ingredients under stirring. Deposit into moulds printed into starch trays and let sit for at least 48 hours at RT. Remove starch powder and polish with oil or wax. Dry at RT and package into airtight pouches

### Example 12

The efficacy of the combination of EGCG and RK as well as of both compounds alone on body weight and adiposity was tested in a 4-week study in C57BL6/J mice fed a high fat, high sucrose diet (n=8-11/group). This model of diet-induced obesity and early type 2 diabetes is widely used to determine the efficacy of anti-obesity compounds.

Male C57BL6/J mice were obtained from Jackson Laboratory (Bar Harbor, ME, USA). Adult mice aged 4 weeks were used in the experiment. Mice were housed individually in plastic cages with bedding and allowed free access to a high fat, high sucrose mouse diet (Kliba # 2154, Klibamuehle, Kaiseraugst, Switzerland) and tap water. The animal rooms were controlled for temperature (24°C), humidity (55%), and light (12-h light-dark cycle). The animals were randomized into four groups. EGCG and RK were administered as feed-ad-mix. Corn cellulose (2% of diet) served as a carrier substance for EGCG and RK as well as a placebo when used alone. Group 1 received placebo, group 2 received EGCG at a dose of 1400 mg/kg body weight (BW)/day, group 3 received RK at a dose of 1400 mg/kg BW/day, and group 4 received the combination of EGCG and RK at a doses of 1400 and 1400 mg/kg BW/day, respectively. Body weight and food intake were determined over the course of the study. All data are expressed as means for animals in each diet group.

Body weight for each treatment group is shown in Table 1. There was no difference in food intake between the groups over the study period. Mice on the control diet consumed the same amount of energy as mice supplemented with EGCG, RK or the combined treatment of EGCG + RK. The decrease in body weight in comparison to the control group caused by each treatment was calculated (data are shown in Table 2). The expected treatment effect of the combined therapy with EGCG and RK was calculated by the sum of the effects exerted by monotherapy with EGCG or RK. The synergistic factor (SF) is defined as the quotient of the observed effect and the expected effect. If SF>1, a synergistic effect was exerted by the combined treatment.

**Table 1**

| | Body weight | | |
|---|---|---|---|
| | Initial (g) | Week 2 (g) | Week 4 (g) |
| Control | 10.6 | 20.4 | 23.6 |
| EGCG(1400mg/kgBW/day) | 11.4 | 17.7* | 21.4* |
| RK(1400 mg/kg BW/day) | 11.5 | 19.0* | 22.1* |
| EGCG + RK (1400 + 1400 mg/kg BW/day) | 11.2 | 14.9* | 18.3* |

| | | | |
|---|---|---|---|
| * significantly different from control (P values less than 0.05 were considered significant) | | | |

**Table 2**

| | Body weight decrease | |
|---|---|---|
| | Week 2 (g) | Week 4 (g) |
| EGCG (1400 mg/kg BW/day) | 2.7 | 2.2 |
| RK (1400 mg/kg BW/day) | 1.6 | 1.5 |
| Expected (EGCG (1400 mg/kg BW/day) + RK (1400 mg/kg BW/day)) | 4.3 | 3.7 |
| Combined treatment EGCG + RK (1400 + 1400 mg/kg BW/day) | 5.5* | 5.3* |
| SF | 1.28 | 1.43 |

| | | |
|---|---|---|
| * significantly different from expected (P values less than 0.05 were considered significant) | | |

When C57BL6/J mice were fed a high fat, high sucrose diet the body weight increased markedly during the 4 week study period. However, dietary supplementation with EGCG or RK resulted in a significant decrease in body weight compared to control mice without supplementation. The combined treatment with EGCG and RK lead to a greater decrease in body weight than exerted by any monotherapy. Moreover, the decrease in body weight due to the combined treatment was significantly greater than the decrease that would be expected by adding the effects of EGCG and RK. Thus, the effect of the combined treatment is synergistic as indicated by a SF>1 for week 2 and week 4.

### Example 13

The efficacy of the combination of EGCG and RK as well as of both compounds alone on body weight and adiposity was tested in a 5-week study in C57BLKS/J db/db mice (n=8-9/group). This model of late type 2 diabetes with severe hyperglycemia and obesity is widely used to determine the efficacy of anti-diabetic and anti-obesity compounds.

Male db/db mice were obtained from Jackson Laboratory (Bar Harbor, ME, USA). Adult mice aged 8 weeks were used in the experiment. Mice were housed individually in plastic cages with bedding and allowed free access to standard rodent food and tap water. The animal rooms were controlled for temperature (24°C), humidity (55%), and light (12-h light-dark cycle). The animals were randomized into four groups. EGCG and RK were administered as feed-ad-mix. Corn cellulose (2% of diet) served as a carrier substance for EGCG and RK as well as a placebo when used alone. Group 1 received placebo, group 2 received EGCG at a dose of 1400 mg/kg BW/day, group 3 received RK at a dose of 1400 mg/kg BW/day, and group 4 received the combination of EGCG and RK at a doses of 1400 and 1400 mg/kg BW/day, respectively. Body weight and food intake were determined over the course of the study. All data are expressed as means for animals in each diet group.

Body weight for each treatment group is shown in Table 3. There was no difference in food intake between the groups over the study period. The decrease in body weight in comparison to the control group caused by each treatment was calculated (data are shown in Table 4). The expected treatment effect of the combined therapy with EGCG and RK was calculated by the sum of the effects exerted by monotherapy with EGCG or RK. The synergistic factor (SF) is defined as the quotient of the observed effect and the expected effect. If SF>1, a synergistic effect was exerted by the combined treatment.

**Table 3**

| | Body weight | | |
|---|---|---|---|
| | Initial (g) | Week 2 (g) | Week 4 (g) |
| Control | 32.2 | 38.7 | 38.4 |
| EGCG (1400 mg/kg BW/day) | 32.2 | 35.7* | 37.7 |
| RK (1400 mg/kg BW/day) | 32.5 | 38.4 | 38.0 |
| EGCG + RK (1400 + 1400 mg/kg BW/day) | 32.4 | 33.7* | 34.3* |

| | | | |
|---|---|---|---|
| *significantly different from control (P values less than 0.05 were considered significant) | | | |

**Table 4**

| | Body weight decrease | |
|---|---|---|
| | Week 2 (g) | Week 4 (g) |
| EGCG (1400 mg/kg BW/day) | 3.0 | 0.8 |
| RK (1400 mg/kg BW/day) | 0.3 | 0.4 |
| Expected (EGCG (1400 mg/kg BW/day) + RK (1400 mg/kg BW/day)) | 3.3 | 1.2 |
| Combined treatment | 5.0* | 4.1* |
| EGCG + RK (1400 + 1400 mg/kg BW/day) | | |
| SF | 1.52 | 3.42 |

| | | |
|---|---|---|
| * significantly different from expected (P values less than 0.05 were considered significant) | | |

The db/db model is widely used to determine the efficacy of anti-diabetic and anti-obesity compounds. As shown in Table 1 these mice rapidly develop severe obesity in early life and thereafter reach a plateau in body weight. Dietary supplementation with EGCG resulted in a significant decrease in body weight at week 2 compared to control mice without supplementation. Treatment with RK did not significantly decrease body weight during the study period. After four weeks of treatment neither EGCG nor RK caused a significant decrease in body weight when used as a monotherapy but the combined treatment with EGCG and RK significantly decreased body weight as compared to the untreated control group. Thus, the combined treatment with EGCG and RK lead to a greater decrease in body weight than exerted by any monotherapy. Moreover, the decrease in body weight due to the combined treatment was significantly greater than the decrease that would be expected by adding the effects EGCG and RK. The combined treatment with EGCG and RK exerted an unexpected synergistic effect on the body weight decrease as indicated by a SF> 1 for week 2 and week 4.

## Claims

1. A nutraceutical composition for the treatment or prevention of obesity or conditions associated with obesity comprising an effective amount of epigallocatechin gallate (EGCG) and 4-(4-hydroxyphenyl)-2-butanone (RK).

2. Composition as in claim 1 comprising EGCG In an amount sufficient to administer to a subject about 0.01 mg to about 60 mg of EGCG per kg body weight and RK In an amount sufficient to administer to a subject about 0.01 mag to about 60 mg of RK per kg body weight.

3. Composition as in claim 1 or 2 which is a pharmaceutical composition.

4. Composition as in claim 3 which is a solid pharmaceutical composition comprising about 10 mg to about 500 mg of EGCG and about 10 mg to about 500 mg of RK in a dosage unit.

5. Composition as in claim 3 which is a solid pharmaceutical composition comprising about 10 mg to about 50 mg of EGCG and about 10 mg to about 50 mg of RK in a dosage unit.

6. Composition as in claim 1 or 2 which is a food or beverage or a supplement composition for a food or beverage.

## Patentansprüche

1. Nutraceutical-Zusammensetzung für die Behandlung oder Vorbeugung von Fettleibigkeit oder mit Fettleibigkeit einhergehenden Leiden, umfassend eine wirksame Menge von Epigallokatechingallat (EGCG) und 4-(4-Hydroxyphenyl)-2-butanon (RK).

2. Zusammensetzung nach Anspruch 1, umfassend EGCG in einer Menge, die ausreicht, einem Patienten ungefähr 0,01 mg bis ungefähr 60 mg EGCG pro kg Körpergewicht zu verabreichen, und RK in einer Menge, die ausreicht, einem Patienten ungefähr 0,01 mg bis ungefähr 60 mg RK pro kg Körpergewicht zu verabreichen.

3. Zusammensetzung nach Anspruch 1 oder 2, bei der es sich um eine pharmazeutische Zusammensetzung handelt.

4. Zusammensetzung nach Anspruch 3, bei der es sich um eine feste pharmazeutische Zusammensetzung umfassend ungefähr 10 mg bis ungefähr 500 mg EGCG und ungefähr 10 mg bis ungefähr 500 mg RK in einer Dosierungseinheit handelt.

5. Zusammensetzung nach Anspruch 3, bei der es sich um eine feste pharmazeutische Zusammensetzung umfassend ungefähr 10 mg bis ungefähr 50 mg EGCG und ungefähr 10 mg bis ungefähr 50 mg RK in einer Dosierungseinheit handelt.

6. Zusammensetzung nach Anspruch 1 oder 2, bei der es sich um ein Nahrungsmittel oder Getränk oder eine Zusatzstoffzusammensetzung für ein Nahrungsmittel oder Getränk handelt.

## Revendications

1. Composition nutraceutique destinée au traitement ou à la prévention de l'obésité ou des affections associées à l'obésité, comprenant une quantité efficace de gallate d'épigallocatéchine (EGCG) et de la 4-(4-hydroxyphényl)-2-butanone (RK).

2. Composition selon la revendication 1, comprenant l'EGCG dans une quantité suffisante pour administrer à un sujet environ 0,01 mg à environ 60 mg d'EGCG par kg de poids corporel et de la RK dans une quantité suffisante pour administrer à un sujet environ 0,01 mg à environ 60 mg de RK par kg de poids corporel.

3. Composition selon la revendication 1 ou 2, **caractérisée en ce qu'**il s'agit d'une composition pharmaceutique.

4. Composition selon la revendication 3, **caractérisée en ce qu'**il s'agit d'une composition pharmaceutique solide comprenant environ 10 mg à environ 500 mg d'EGCG et environ 10 mg à environ 500 mg de RK dans une unité de dose.

5. Composition selon la revendication 3, **caractérisée en ce qu'**il s'agit d'une composition pharmaceutique solide comprenant environ 10 mg à environ 50 mg d'EGCG et environ 10 mg à environ 50 mg de RK dans une unité de dose.

6. Composition selon la revendication 1 ou la revendication 2, **caractérisée en ce qu'**il s'agit d'un aliment ou d'une boisson ou d'une composition de complément pour un aliment ou une boisson.
